# EUROPÄISCHE PATENTSCHRIFT

(11) **EP 3 204 350 B1**
(45) Veröffentlichungstag und Bekanntmachung des Hinweises auf die Patenterteilung: **21.03.2018**
(21) Anmeldenummer: 16805139.9
(22) Anmeldetag: 02.12.2016
(51) Int. Cl.: C07C 68/04

(54) **VERFAHREN UND VORRICHTUMG ZUR KONTINUIERLICHEN HERSTELLUNG VON ORGANISCHENCARBONATEN AUS CO2**
METHOD AND DEVICE FOR THE CONTINUOUS PRODUCTION OF ORGANIC CARBONATES FROM CO2
PROCEDE ET DISPOSITIF DE FABRICATION CONTINUE DE CARBONATES ORGANIQUES A PARTIR DE CO2

(30) Priorität: 02.12.2015 EP 15003431
(43) Veröffentlichungstag der Anmeldung: 16.08.2017
(73) Patentinhaber: AIT Austrian Institute of Technology GmbH, 1220 Wien (AT)
(72) Erfinder: LAZAROVA, Zdravka, 1200 Wien (AT)
(74) Vertreter: Sonn & Partner Patentanwälte
(86) Internationale Anmeldenummer: PCT/EP2016/079564
(87) Internationale Veröffentlichungsnummer: WO 2017/093472

(56) Entgegenhaltungen:
- US-A1- 2011 196 167
- H.J.LEE; W. JOE; J. C. JUNG; I. K. SONG: "Direct synthesis of dimethyl carbonate from methanol and carbon dioxide over Ga2O3-CeO2-ZrO2 catalysts prepared by a single-step sol-gel method: Effect of acidity and basicity of the catalysts", IM KOREAN J. CHEM. ENG., Bd. 29, Nr. 8, 2012, Seiten 1019-1024, XP035093277, DOI: 10.1007/s11814-012-0017-0 in der Anmeldung erwähnt

## Beschreibung

Die vorliegende Erfindung betrifft ein Verfahren und eine Vorrichtung zur Herstellung organischer Carbonate (R¹O₂)CO durch katalysierte Reaktion von entsprechenden Alkoholen R¹OH mit Kohlendioxid.

Aufgrund ihrer vorteilhaften physikochemischen Eigenschaften finden organische Carbonate vielseitige Anwendung in verschiedenen Gebieten mit großem Marktpotential: als umweltfreundliche Lösungsmittel (Ersatz von Ketonen und Estern) in der Produktion von Agrochemikalien und Pharmazeutika, in der Lackindustrie, Erdgasaufbereitung und Kosmetik; als Reagenzien in der Synthese von Kunststoffen wie Polycarbonaten, Urethanen und Isocyanaten (Ersatz von toxischen Chemikalien für Carbonylierung und Methylierung wie Phosgen, Dimethylsulfat, Methylhalogeniden).

Derzeit zeichnen sich weitere vielversprechende Ansätze ab: so stellt Dimethylcarbonat (DMC) eine umweltfreundliche Alternative zum weitverbreiteten MTBE (Methyl-tertiär-butylether) als Antiklopfmittel für Treibstoffe (erhöht die Oktanzahl und reduziert CO-, SOx-, NOx- Ruß-Emissionen) dar; eine Reihe von Carbonaten (wie beispielsweise Ethylcarbonat EC, DMC, Propylcarbonat PC, Diethylcarbonat DEC und Ethylmethylcarbonat EMC) erweisen sich als bevorzugte Elektrolytbestandteile der Lithiumbatterien für die modernen Hybrid- und Elektrofahrzeuge.

Die industriellen Verfahren zur Herstellung von organischen Carbonaten basieren auf Synthesemethoden, bei denen vor allem Alkohole durch chemische Katalyse in Carbonate umgewandelt werden. In der Praxis werden beispielsweise zur DMC-Produktion unterschiedliche Syntheserouten angewendet, wie dem Review von Santos et.al. [Bruno A.V. Santos, Viviana M.T.M. Silva, Jose M., ChemBioEng Rev 2014, 1, No 5, 214-229] zu entnehmen ist.

Der älteste Prozess, die Phosgenierung von Methanol nach der Reaktion

2MeOH + Phosgen ↔ DMC + 2HCl (Pyridin als Katalysator) (1)

nützt als Ausgangsprodukt das sehr toxische Gas Phosgen und als Katalysator das gesundheitsschädliche und leichtentzündliche Pyridin; während des Prozesses werden stark korrosive Reaktionsprodukte gebildet. Marktstudien zeigen, dass trotz der hohen Umsatzraten dieser Prozess nicht mehr oder nur begrenzt angewendet wird.

Um das Phosgen als Reagenz zu vermeiden, wurde eine Reihe von alternativen Prozessen entwickelt. Der Bekannteste davon beruht auf der Oxy-Carbonylierung von Alkoholen mittels CO und Sauerstoff in Gegenwart von Cu-Salzen als Katalysatoren (ENIChem Synthesis, Italien):

2MeOH +CO + O₂ ↔ DMC + H₂O (2)

Die Entwicklung basierend auf der US 4,218,391 B1 wurde später verbessert, um die Nachteile der ursprünglichen Technologie zu beheben. So wird z.B. der Verlust an Chlor aus dem Katalysator mittels in situ Zusatz von Salzsäure kompensiert (US 5,686,644 B1). Neben den Korrosionsproblemen besteht bei diesem Verfahren auch ein Explosionsrisiko.

Eine Modifikation der oben beschriebenen Variante ist die Carbonylierung von Methylnitrit, wie sie beispielsweise von der Ube Chemical Industries eingesetzt wird. Der Unterschied liegt darin, dass die Oxidation von Methanol nicht mit Sauerstoff sondern mit Stickstoffoxiden durchgeführt wird, um das Explosionsrisiko zu vermeiden (US 5,214,185 B1, US 5,631,396 B1). Die Reaktion verläuft in zwei nach einander folgenden Schritten mit Pd/Cu-Salzen als Katalysator

2MeOH + N₂O₃ ↔ 2MeNO₂ + H₂O (3)

2MeNO₂ + CO ↔ DMC + NO (4),

wobei das zuerst gebildete Methylnitrit mittels CO carbonyliert und in DMC umgewandelt wird. Nachteilig sind bei diesem Verfahren die Nutzung von CO, die weiterhin bestehenden Korrosionsprobleme sowie der Einsatz von teuren Katalysatoren.

Eine niedrigere Toxizität zeigt ein später entwickelter Prozess, bei dem CO durch Harnstoff ersetzt wird (US 2006/0047136 A1). In zwei Reaktionsschritten (mit Metall-Oxiden als Katalysator) bildet sich neben NH₃ und Methylcarbamat (Zwischenprodukt) auch DMC:

MeOH + (NH₂)₂O ↔ MeNH₂O₂ (Methylcarbamat) + NH₃ (5)

MeOH+MeNH₂O₂ ↔ DMC + NH₃ (6)

Die Nachteile dieses Verfahrens sind eine niedrige Ausbeute, die relativ hohe Temperatur von über 150°C sowie das hohe MeOH/Harnstoff-Verhältnis.

Zurzeit wird DMC von anderen großen Firmen wie General Electric Plastics (jetzt SABIC), Texaco Inc., Mitsubishi Chemicals Corp., BASF hergestellt.

Bei allen erwähnten Methoden werden meistens mehrstufige Verfahren angewendet, bei denen viele unerwünschte Nebenprodukte entstehen, außerdem entsprechen sie zumeist nicht den Prinzipien einer umweltfreundlichen Chemie. Als Ausgangsprodukte werden toxische Chemikalien wie z.B. Kohlenmonoxid oder Nitritoxid benutzt; das Gemisch Methanol/NO/O₂ ist potentiell explosiv. Die Selektivität zu Estern ist begrenzt, die Nebenprodukte wie z.B. Methylchlorid verursachen eine starke Metallkorrosion.

Die potentiell attraktivste Variante der Synthese von organischen Carbonaten bietet die direkte chemische Reaktion von Alkoholen mit Kohlendioxid an. Dadurch kann der technologische Prozess einfacher und ökonomischer gestaltet werden. Ein zusätzlicher ökologischer Vorteil entsteht durch die Nutzung von CO₂ als Rohstoff: das Kohlendioxid, welches vor allem bei Verbrennungsprozessen entstehet, wird als C1 Baustein in der Produktion eingesetzt, dadurch nicht in die Atmosphäre abgelassen und seine nutzbringende Verwendung kann somit auch den unerwünschten Treibhausgaseffekt reduzieren.

Aus diesen Gründen hat es nicht an Versuchen gefehlt, Kohlendioxid mit Alkoholen in Gegenwart unterschiedlicher Katalysatoren zu Carbonaten umzusetzen. In den letzten Jahren sind auf diesem Gebiet eine Reihe von Patentanmeldungen veröffentlicht worden und zahlreich wissenschaftliche Artikeln erschienen.

Schwerpunkt bei allen neuen Entwicklungen ist die Überwindung von kinetischen und thermodynamischen Limitierungen durch 1) den Einsatz neuer Katalysatoren (organischen und anorganischen), 2) den in-situ Wasserentzug mittels zusätzlicher Chemikalien und 3) den Einsatz von zusätzlichen chemischen Promotoren und Co-Katalysatoren. Vorangetrieben wird dabei die Entwicklung von sowohl homogenen als auch heterogenen Katalyseprozessen.

In der JP 2012 162523 A ist die Herstellung von Carbonaten aus Kohlendioxid und dem entsprechenden Alkohol in Gegenwart von organischen Nitril-Verbindungen als Katalysatoren (2-Cyanopyridin, 2-Cyanopyrazin, 2-Cyanopyrimidin, 2-Thiophenecarbonitril, 2-Furancarbonitril und anderen) beschrieben, wobei eine zusätzliche Bildung von Amiden beobachtet wird.

Im Korean J. Chem. Eng. (H.J.Lee , W. Joe, J. C. Jung, I. K. Song Vol.29, Iss. 8, 1019-1024, 2012;, Seoul National University) ist ein Verfahren zur Herstellung organischer Carbonate aus Kohlendioxid und dem entsprechenden Alkohol in Gegenwart von anorganischen Katalysatoren wie Ga₂O₃-CeO₂-ZrO₂ beschrieben.

Die CN 1264698/2000 A beschreibt ein Verfahren zur Herstellung von organischen Carbonaten aus Kohlendioxid und dem entsprechenden Alkohol mittels anorganischer Katalysatoren wie Kaliumcarbonat, allerdings in Kombination mit toxischen Promotern, wie Methyliodid. Die Verwendung von Kaliumsalzen in Gegenwart von Co-Katalysatoren (Epoxydverbindungen) wird auch in der CN 103508894/2015 A beschrieben.

Aus der CN 1131660/1996 A ist beispielsweise ein Verfahren zur direkten Synthese von DMC mittels Mg als Katalysator bekannt, bei dem als Zwischenprodukt Mg-Formiat gebildet wird.

In der KR 0129935/2014 A ist ein Verfahren zur Herstellung von Dimethylcarbonat aus Methanol und CO₂ in Gegenwart eines Trägerkatalysators beschrieben. Der Träger besteht aus einem Metalloxid (mindestens Ceriumoxid oder Zinkoxid); der Katalysator enthält zumindest Nickeloxid oder Zirkoniumoxid.

Die US 2011/196167 A beschreibt ein Verfahren zur Herstellung von DMC aus Methanol und Kohlendioxid unter Verwendung eines heterogenen Katalysators, welcher Katalysator sowohl Säurestellen als auch Basenstellen vorsieht.

Alle bekannten Verfahren zur direkten Herstellung von organischen Carbonaten aus Kohlendioxid und dem entsprechenden Alkohol verwenden entweder teure Katalysatoren oder Katalysatoren in Verbindung mit teuren oder giftigen Promotoren, oder Co-Katalysatoren. Zusätzlich nachteilig ist der Umstand, dass es sich dabei zumeist um wasserempfindliche Katalysatoren handelt. Um die Schädigung des Katalysators zu vermeiden, muss somit das während der Redaktion als Nebenprodukt entstehende Wasser aus dem Reaktionssystem ständig entfernt werden. Für diesen Zweck werden zusätzliche Reagenzien als Wasserfänger benutzt. Die JP 07033715/1995 A beschreibt z.B. die Verwendung von Orthoestern, die bei der Reaktion mit Wasser verbraucht werden. Beschrieben werden auch Varianten dieses Verfahrens, bei denen ein Molekularsieb angewendet wird (CN 1368495/2002 A).

Alle beschriebenen Reagenzien zur Entfernung von Wasser verbrauchen sich jedoch relativ schnell oder erfordern aufwendige Schritte zu ihrer Regeneration. Außerdem beeinflussen sie die Selektivität der Synthese durch Bildung von Nebenprodukten, die auch zur Verunreinigung des Produktes führen.

Aus den oben beschriebenen Gründen ist die auf CO₂ basierende direkte Synthese von organischen Carbonaten, insbesondere von DMC, immer noch nur ein Forschungsobjekt. Es ist bis jetzt keine industrielle Anlage bekannt, die beispielsweise DMC kontinuierlich aus CO₂ als Rohstoff produziert.

Die Aufgabe der vorliegenden Erfindung besteht daher darin, ausgehend vom oben dargelegten Stand der Technik eine kostengünstige und umweltfreundliche Synthese von organischen Carbonaten, basierend auf der katalysierten Reaktion zwischen Kohlendioxid und dem bzw. den entsprechenden Alkohol(en), bereitzustellen.

Eine weitere Aufgabe der vorliegenden Erfindung besteht darin, ein solches Verfahren kontinuierlich zu gestalten und eine dazugehörige Vorrichtung (Anlage) zur Herstellung organischer Carbonate (einschließlich CO₂-Aufbereitung, chemische Reaktion, Phasen-Separation, Produkt-Reinigung, Recycling der unverbrauchten Ausgangsprodukte und der Regenerierung verbrauchter Chemikalien) zur Verfügung zu stellen.

Demgemäß wird erfindungsgemäß ein Verfahren zur Herstellung organischer Carbonate (R¹O₂)CO durch katalysierte Reaktion von entsprechenden Alkoholen R¹OH mit Kohlendioxid zur Verfügung gestellt, wobei die Ausgangsprodukte für die Reaktion neben ROH und Kohlendioxid auch das Endprodukt (R¹O₂)CO umfassen, wobei R¹ ein gerad- oder verzweigtkettiger Alkylrest mit 1-4 Kohlenstoffatomen ist und als Katalysatoren Verbindungen der Formel Alkali₂CO₃ verwendet werden, wobei Alkali ein Element ausgewählt aus der Gruppe Lithium, Natrium, Kalium, Rubidium, Cäsium darstellt, vorzugsweise K₂CO₃ und Cs₂CO₃ sowie Gemische aus den beiden, besonders bevorzugt K₂CO₃. So umfassen beispielsweise die Ausgangsprodukte für die Herstellung von DMC nach dem erfindungsgemäßen Verfahren bereits DMC sowie Methanol, CO₂ sowie eine Base (K₂CO₃). Die Base fungiert dabei sowohl als Katalysator als auch als in situ Wasserfänger; das System beinhaltet keine zusätzlichen Promotoren. Die direkte Reaktion zwischen CO₂ und einem Alkohol findet in Gegenwart einer bestimmten Menge des bei Verwendung des jeweiligen Alkohols zu erwartenden Endprodukts und nur noch einer basischen Verbindung als Katalysator statt. Im Falle von DMC wird ein anorganisches Carbonat (K₂CO₃) als Katalysator verwendet, theoretisch könnte ebenso auch eine organische Base verwendet werden. Da Alkalicarbonate im Reaktionssystem in Gegenwart von Kohlendioxid und Wasser entsprechend der Reaktion

Alkali₂CO₃ + CO₂ + H₂O ⇆ 2 AlkaliHCO₃ (9)

in Alkalibicarbonate übergehen, sind die entsprechenden Alkalibicarbonate ebenfalls als Katalysatoren geeignet.

In der anschließenden Tabelle werden die Ergebnisse der Löslichkeitsversuche einzelner Katalysatoren in unterschiedlichen Alkoholen (jeweils gemessen bei 20°C) dargestellt.

| | | | |
|---|---|---|---|
| K₂CO₃ | in Methanol | 17,01 | [g/l] |
| K₂CO₃ | in Ethanol | 0,28 | [g/l] |
| Cs₂CO₃ | in Methanol | 56,95 | [g/l] |
| Cs₂CO₃ | in Ethanol | 28,9 | [g/l] |
| Cs₂CO₃ | in Propanol | 18,8 | [g/l] |
| Cs₂CO₃ | in n-Butanol | 11,69 | [g/l] |

Die Ergebnisse zeigen, dass sowohl K₂CO₃ als auch Cs₂CO₃ (um zwei erfindungsgemäß zum Einsatz kommende Katalysatoren in beispielhafter Form zu nennen) in den erfindungsgemäß verwendeten Alkoholen relativ gut löslich sind und es zur Verbesserung der Umsetzungen bei Verwendung einer heterogenen Katalyse daher des Zusatzes eines weiteren (löslickeitsreduzierenden) Lösungsmittels bedarf.

Weiters wurden für einzelne Katalysatoren Löslichkeitsversuche in dem erfindungsgemäß bevorzugt hergestellten DMC durchgeführt, auch hier bei 20°C. Dabei zeigte sich, dass im organischen Carbonat DMC deutlich weniger Katalysator löslich ist als im zur Herstellung von DMC verwendeten Alkohol (Methanol).

| | | |
|---|---|---|
| K₂CO₃ | in DMC 0,26 | [g/l] |
| Cs₂CO₃ | in DMC 0,09 | [g/l] |

Schließlich wurden für diese Katalysatoren noch Löslichkeitsversuche in dem erfindungsgemäß verwendeten Gemisch aus Endprodukt und Alkohol (hier DMC und Methanol, 85:15 %-wt, Temperatur 20°C) durchgeführt. Aus den nachstehend angegebenen Ergebnissen ist zu sehen, dass auch die Löslichkeit von Cs₂CO₃ im Alkohol (Methanol) mittels Zugabe des Endprodukts zum Alkohol um Größenordnungen gesenkt werden konnte, aber doch noch über jener von K₂CO₃ blieb.

| | | |
|---|---|---|
| K₂CO₃ | in DMC/MeOH | 0,32 [g/l] |
| Cs₂CO₃ | in DMC/MeOH | 8,91 [g/l] |

K₂CO₃ ist daher der bevorzugte erfindungsgemäß zum Einsatz kommende Katalysator.

Gemäß einer bevorzugten Ausführungsform der vorliegenden Erfindung ist vorgesehen, dass die Ausgangsprodukte 30 bis 90 %-wt, vorzugsweise 65 bis 85 %-wt, an Endprodukt (R¹O₂)CO umfassen.

Günstig ist ebenfalls, wenn die verwendeten Katalysatoren zusätzlich zu ihrer katalytischen Wirkung auch wasseraufnehmend wirken; unter bestimmten Reaktionsbedingungen wirkt der verwendete Katalysator nämlich gleichzeitig als Kohlendioxid- und Wasserfänger.

Gemäß einer bevorzugten Ausführungsform der vorliegenden Erfindung wird das Ausgangsprodukt CO₂ der Reaktion als Gas oder als Flüssigkeit zugeführt.

Vorzugsweise ist vorgesehen, dass die Reaktion kontinuierlich oder diskontinuierlich durchgeführt wird.

Gemäß einer weiteren bevorzugten Ausführungsform der vorliegenden Erfindung wird die Reaktion in einem Strömungsreaktor oder in einer Serie von Reaktoren durchgeführt.

Allgemein ist erfindungsgemäß als bevorzugt vorgesehen, wenn die Reaktion bei einer Temperatur von 60-140°C, bevorzugt 90-110°C, durchgeführt wird.

Günstig ist dabei auch, wenn die Reaktion bei einem Druck von 20-120 bar, bevorzugter 30-50 bar, am bevorzugtesten 70-90 bar durchgeführt wird.

Eine weitere Ausführungsform der vorliegenden Erfindung sieht vor, dass als Alkohol Methanol, Ethanol, Propanol oder Butanol eingesetzt wird.

Erfindungsgemäß hat sich als günstig erwiesen, wenn bei den Ausgangsprodukten die Menge an Katalysator im Verhältnis zum eingesetzten Alkohol 0,02-1,0 Moläquivalente beträgt und/oder wenn bei den Ausgangsprodukten die Menge an organischem Carbonat im Verhältnis zum eingesetzten Alkohol 0,5-5,0 Moläquivalente beträgt und/oder wenn bei den Ausgangsprodukten die Menge an CO₂ im Verhältnis zum eingesetzten Alkohol 0,5-7,5 Moläquivalente beträgt.

Vorzugsweise erfolgt beim erfindungsgemäßen Verfahren die Abtrennung des Endprodukts durch Membranpervaporation oder Dampfpermeation, oder als Hybridseparation in Kombination mit Destillation erfolgt.

Gemäß einer besonders bevorzugten Ausführungsform der vorliegenden Erfindung ist vorgesehen, dass der eingesetzte Alkohol Methanol (MeOH) und das Endprodukt Dimethylcarbonat (DMC) ist. Dabei ist günstig, wenn als Ausgangsprodukte für die Reaktion DMC und MeOH in einem azeotropen Mischungsverhältnis vorliegen.

So umfassen beispielsweise die Ausgangsprodukte für die Herstellung von DMC nach dem erfindungsgemäßen Verfahren bereits DMC sowie Methanol, CO₂ sowie eine Base. Die Base fungiert dabei sowohl als Katalysator als auch als in situ Wasserfänger; das System beinhaltet keine zusätzlichen Promotoren. Die direkte Reaktion zwischen CO₂ und einem Alkohol findet in Gegenwart einer bestimmten Menge des bei Verwendung des jeweiligen Alkohols zu erwartenden Endprodukts und nur noch einer basischen Verbindung statt. Im Falle von DMC kann ein anorganisches Carbonat wie z.B. K₂CO₃ oder eine organische Base verwendet werden. Unter bestimmten Reaktionsbedingungen kann der verwendete Katalysator gleichzeitig als Kohlendioxid- und Wasserfänger fungieren.

Bei dem erfindungsgemäßen Verfahren werden daher keine toxischen Promotoren, wie CH₃I, oder zusätzlichen in situ wasserbindenden Chemikalien, und auch keine Co-Katalysatoren eingesetzt.

Die Gegenwart des bei Verwendung des jeweiligen Alkohols zu erwartenden Endprodukts schon als Ausgangsprodukt zum Zeitpunkt des Reaktionsbeginns beeinflusst wesentlich das gegenseitige Zusammenwirken der anderen Ausgangsprodukte (Alkohol, CO₂, Base) und ermöglicht dadurch die zusätzliche Bildung von Endprodukt (organisches Carbonat). So hat beispielsweise DMC die Eigenschaft, im Vergleich zu Methanol mehr CO₂ zu absorbieren und weniger Katalysator-Salze zu lösen.

Das Endprodukt (organisches Carbonat) stellt daher einerseits ein geeignetes Lösungsmittel für die verwendeten Ausgangsprodukten dar, andererseits trägt das organische Carbonat dazu bei, das bei der Synthesereaktion als Nebenprodukt gebildete Reaktionswasser aus der flüssigen in die feste Phase (Katalysator-Salz) hineinzudrängen. Das führt dazu, dass das Reaktionswasser von dem basischen Salz aufgenommen und aus dem flüssigen Reaktionsgemisch entfernt wird. Auf diese Weise wird das Reaktionsgleichgewicht ohne Zugabe von zusätzlichen Chemikalien weiter in Richtung des Endprodukts verschoben.

Die Synthesereaktion zeichnet sich durch einen sehr hohen Selektivitätsgrad aus, es wurden weder in der flüssigen Phase noch im Abgas Nebenprodukte analytisch identifiziert.

Außerdem hat die Verwendung des Endprodukts (organisches Carbonat) als Bestandteil der Ausgangsprodukte beim Reaktionsstart eine deutlich positive Wirkung auf die nachfolgende Phasenseparation und die Produkt-Feststoff-Abtrennung.

Das hier beschriebene Verfahren zur Herstellung von organischen Carbonaten, insbesondere DMC, stellt eine umweltfreundliche CO₂-basierte Synthese dar, bei welcher eine maximal mögliche Ausbeute ohne Zugabe von toxischen und/oder teuren Katalysatoren und Promotoren und Additiven erreicht wird.

Unter einem organischen Carbonat wird gemäß der vorliegenden Erfindung ein Ester der Kohlensäure der allgemeinen Formel 7 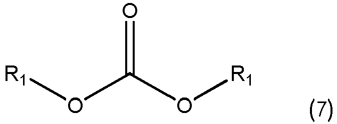 verstanden, bei dem R₁ ein gerad- oder verzweigtkettiger Alkylrest mit 1-4 Kohlenstoffatomen ist, Beispiele für solche Alkylreste sind Methyl, Ethyl, Propyl, i-Propyl, n-Butyl, sekundäres-Butyl und tertiäres Butyl.

Besonders bevorzugt hergestellte organische Carbonate sind Dimethylcarbonat, Diethylcarbonat und Dipropylcarbonat.

Der erfindungsgemäß verwendbare Alkohol ist demgemäß die Verbindung der Formel (8)

R₁-OH (8)

bei der R₁ einen Rest entsprechend obiger Definition darstellt.

Weitere Ziele, Merkmale, Vorteile und Anwendungsmöglichkeiten der erfindungsgemäßen Verfahren und der (vorzugsweise kontinuierlich betriebenen) Anlage zur Herstellung von organischen Carbonaten ergeben sich aus der nachfolgenden Beschreibung von Ausführungsbeispielen anhand der Zeichnungen. Dabei bilden alle beschriebenen und/oder bildlich dargestellten Merkmale für sich oder in beliebiger Kombination den Gegenstand der Erfindung, unabhängig von der Zusammenfassung in einzelnen Ansprüchen oder deren Rückbeziehung.

Unter Bezugnahme auf die nachfolgenden nicht-limitierenden Beispiele werden unterschiedliche verfahrenstechnische Varianten zur vorzugsweise kontinuierlich ablaufenden Synthese von organischen Carbonaten erläutert. Erfindungsgemäß werden zwei Ausführungsformen des Synthesereaktors in Abhängigkeit von der Führung der Fluidströme berücksichtigt, nämlich Variante I als Rohrreaktor mit Etagenrührer und Variante II als Festbettreaktor (befüllt mit Katalysator, immobilisiert auf Träger/n).

Variante I: Im ersten Fall befindet sich das Katalysator-Wasserentzugsreagenz in Pulverform; und der andere Teil der Ausgangsprodukte (mit Ausnahme des Kohlendioxids) stellt eine fest-flüssige Suspension dar, wobei die flüssige Phase aus Methanol oder Carbonat, oder aus einem Methanol-Carbonat Gemisch bestehen kann. Nach dem Vordispergieren des festen Stoffes bzw. Homogenisieren der Suspension wird die Mischung in den Rohrreaktor eingeführt, wo sie sich mit dem zugeleiteten CO₂-Strom vermischt.

Variante II: Im Falle des Festbettreaktors werden die flüssigen Ausgangsprodukte, der Alkohol (z.B. Methanol) und das organische Carbonat (z.B. DMC) und das CO₂ separat oder zusammen vorvermischt und in den mit immobilisiertem Katalysator befüllten Rohrreaktor eingeleitet. Das Kohlendioxid kann sowohl im gasförmigen als auch im flüssigen Zustand in den Reaktor eingeleitet werden. Der Vorteil von CO₂-Gas besteht darin, dass es aus unterschiedlichen Industriequellen mit weniger Aufwand und günstig zur Verfügung gestellt werden kann, der Vorteil von CO₂-flüssig ist die höhere Dichte (Dichtedifferenz >200-mal: Gas - 2 kg/m³ gegenüber flüssig- 450 kg/m³), die zulässt, beim gleichen Volumina größere CO₂ -Mengen zu fördern.

Die direkte Reaktion zwischen Methanol und CO₂ verläuft bevorzugt unter folgenden Verfahrensbedingungen:
- Temperaturbereich von 60-140°C, bevorzugt 90-110°C,
- Druckbereich von 20-120 bar, bevorzugt 30-90 bar, noch bevorzugter 70-90 bar,
- Reaktionszeiten 30-240 Minuten, bevorzugt 60-180 Minuten,
- Gewichtsverhältnis der Ausgangsprodukte und des Katalysators:
   - MeOH: 6-25 %-wt, bevorzugt 7-17 %-wt
   - DMC: 30-90 %-wt, bevorzugt 65-85 %-wt bzw. 0,5-5 Äquivalente (bevorzugt 2-4 Äquivalente, bezogen auf Methanol)
   - Katalysator-Wasserfänger: K₂CO₃: 1-35 %-wt, bevorzugt 2-5 %-wt bzw. 0,02-1,0 Äquivalente (bevorzugt 0,05-0,2 Äquivalente bezogen auf Methanol),
   - CO₂: 5-45 %-wt, bevorzugt 15-35 %-wt bzw. 0,5-7,5 Äquivalente (bevorzugt 1,5-4,5 Äquivalente bezogen auf Methanol),

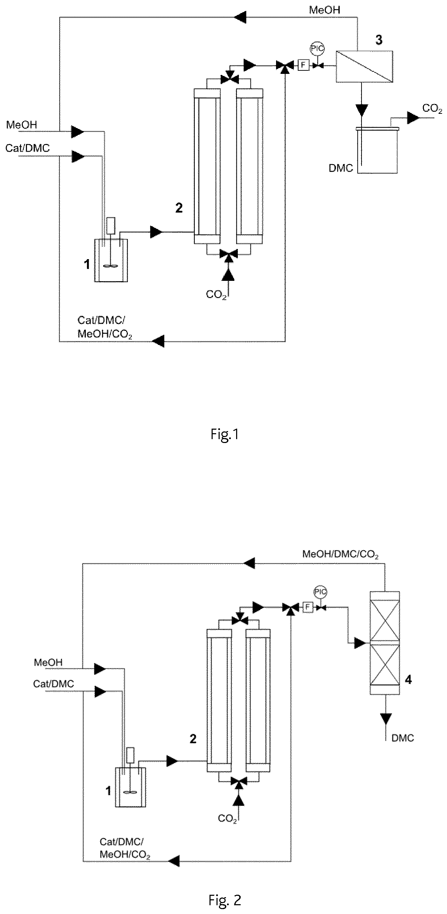

In einer Ausführungsform des erfindungsgemäßen Verfahrens (Fig.1), bei welcher der Katalysator in Pulverform vorliegt, werden Alkohol, das entsprechende organische Carbonat und der feste Katalysator einem geschlossenen Vormischtank **1** kontinuierlich zugeführt, homogen intensiv gerührt und auf 50-60°C vorgeheizt. Die vorerhitzte Suspension wird dann in den nachfolgenden Rohrreaktor **2** geleitet, wo sie mit dem zuströmenden CO₂ unter Druck (40-80 bar) vermischt und weiter bis auf 90-110°C erhitzt wird. Das CO₂ kann sich im gasförmigen oder flüssigen, oder superkritischen Zustand befinden. Das Reaktionsgemisch durchläuft den Rohrreaktor, wobei es entlang des Reaktors weiter gerührt wird. In Abhängigkeit von den gewünschten Verweilzeiten, kann das Reaktorsystem aus einem oder mehreren Reaktoren bestehen. Durch eine gezielte Auswahl der Fluidströme können die Verhältnisse der Massen/Stoff/Volumen-Ströme variiert und entsprechend angepasst werden.

Im Laufe der Reaktion, die unter erhöhtem Temperatur und Druck verläuft, bildet sich neues organisches Carbonat und seine Konzentration im Reaktionsgemisch steigt an. Die angereicherte flüssige Phase kann im Kreis geführt werden, um einen oder mehrere erneute Zyklen im Reaktor zu durchlaufen, bis eine bestimmte Umsetzung stattgefunden hat. Im Falle des Suspensionsreaktors wird der feste pulverförmige Katalysator bei der Kreisführung durch einen Filter zurückgehalten.

Kontinuierlich werden Alkohol, organisches Carbonat und Kohlendioxid zugeführt. Das Verhältnis der zugeführten, abgeführten und zurückgeführten Massenströme bzw. deren Fließgeschwindigkeiten wird so eingestellt, dass im Reaktor der effiziente kontinuierliche Ablauf der Synthesereaktion gewährleistet ist.

Das Reaktionsgemisch wird nach der Reaktion weiter behandelt, um das reine Produkt abzutrennen und unverbrauchte Ausgangsprodukte zurückzuführen. Für diesen Zweck können unterschiedliche Separationsvarianten mit der Synthese kombiniert werden:

### Variante I. Kontinuierliche Synthese mittels Festbett- oder Suspensions-Reaktors, gekoppelt mit Separationsmodul/en zur Pervaporation des Reaktionsgemisches

Ein Teil des Produktstroms wird auf 1-10 bar entspannt und mittels Pervaporations-Membranmodulen weiter behandelt (Fig.1). Das Permeat besteht im Wesentlichen aus Methanol und kann rückgeführt werden. Das Retentat enthält DMC und Reste von CO₂, welche Reste in einer Gasfalle abgetrennt und dem Prozess wieder zugefügt werden können.

### Variante II. Kontinuierliche Synthese mittels Festbett- oder Suspensions-Reaktors, gekoppelt mit einer Destillationsanlage zur Trennung des Reaktionsgemisches

Der Unterschied zu Variante I besteht in der Kombination der Synthese mit einer destillativen Behandlung des Produktgemisches (Fig.2). Ein Teil des Produktstroms wird auf Umgebungsdruck entspannt und destillativ abgetrennt. Reines DMC fällt dabei als Sumpfprodukt an, während ein azeotropes Gemisch aus Methanol und DMC sowie restliches CO₂ als Kopfprodukt anfällt. Dieses Kopfprodukt kann ohne zusätzliche Spaltung des Azeotrops wieder dem Prozess zugefügt und als Ausgangsprodukt benutzt werden.

### Variante III: Kontinuierliche Synthese mittels Festbett- oder Suspensions-Reaktors, gekoppelt mit einer kombinierten Destillations-Membran-Trennung des Reaktionsgemisches

Der Unterschied zu Varianten I und II besteht darin, dass das Kopfprodukt aus der Destillation weiter mittels Membran-Dampfpermeation behandelt wird, wobei das MeOH/DMC/CO₂ Gemisch getrennt und das Azeotrop gespalten wird. MeOH permeiert durch die Membran und kann erneut in den Prozess eingeführt werden. Reines DMC fällt im Retentat an.

### Beispiel 1: Synthese von DMC unter Verwendung eines kontinuierlichen Suspensions-Reaktors

Eine Übersicht der Ausgangsparameter des Reaktionsgemisches und der entsprechenden Versuchsergebnisse bei der Synthese von DMC unter Verwendung eines kontinuierlichen Suspensionsreaktors ist in **Tabelle 1** zu sehen. Umsatzzuwächse sind in der Tabelle als ΔDMC [g/L] angegeben. Versuch Nr. 1 ist der Blindversuch ohne Katalysatorzugabe. Die Versuche mit dem Suspensionsreaktor dauerten zwischen 6 und 12 h; die mittlere Verweilzeit im Reaktor ist mit **τ** bezeichnet. Die Probenahme erfolgte nach jeweils 2 Stunden. Die Tabelle 1 beinhaltet Ergebnisse von Experimenten durchgeführt bei unterschiedlichen Prozessbedingungen.

**Tabelle 1. Übersicht der Versuchsergebnisse unter Verwendung eines kontinuierlichen Suspensions- Reaktors**

| **Nr.** | **K-Salz°** [%wt] | **DMC°** [%wt] | **MeOH°** [%wt] | **CO₂**° [g/min] | **τ** [min] | **Δ DMC** [g/L] |
|---|---|---|---|---|---|---|
| 1 | 0 | 88,0 | 12 | 4,0 | 188 | 6 |
| 2 | 1,0 | 87,0 | 12 | 3,5 | 113 | 19 |
| 3 | 1,5 | 88,5 | 10 | 3,5 | 145 | 27 |
| 4 | 1,5 | 88,5 | 10 | 4,0 | 150 | 43 |
| 5 | 1,5 | 83,5 | 15 | 4,0 | 186 | 46 |
| 6 | 3,0 | 76,0 | 21 | 4,0 | 214 | 69 |

Relevante DMC-Zuwächse sind ab einer K-Salz-Konzentration von 1,5 %-wt zu verzeichnen. Bei einer K-Salz Konzentration von 1,5 %-wt und annähernd gleichen Ausgangsverhältnissen von DMC, MeOH und CO₂ liegen die erzielten DMC-Umsatzzuwächse im Tagesmittel zwischen 27 g/L und 46 g/L. Das beste Ergebnis wurde mit einer höheren K-Salz-Konzentration von 3,0 %-wt erzielt (Versuch 6). Bei diesem Versuch ist auch der MeOH-Anteil entsprechend erhöht, um die Suspension mit dem höheren Feststoffanteil besser transportieren zu können.

Auf Grundlage der Ergebnisse von Versuch 6 wurde eine Massenbilanz ermittelt, die als Ausgangspunkt für weitere Optimierung der Suspensionsbetriebsweise dienen kann:

| | |
|---|---|
| Feed: | 10 g/h K-Salz + 86 g/h MeOH + 504 g/h DMC + 238 g/h CO₂ |
| Produkt: | 10 g/h K-Salz + 72 g/h MeOH + 540 g/h DMC + 216 g/h CO₂ |

Unter den vorliegenden Reaktionsbedingungen bedeutet das eine DMC-Massenzunahme von 36 g/h bei gleichzeitiger MeOH-Abnahme von 14 g/h. Dabei werden 22 g/h CO₂ benötigt.

### Beispiel 2: Synthese von DMC unter Verwendung eines kontinuierlichen Festbett-Reaktors

Für die Versuche mit dem Festbettreaktor wurde der K-Salz-Katalysator auf Al₂O₃-Kugeln immobilisiert. In einem Rohrreaktor wurden 410 g Kugeln mit einem Katalysatoranteil von 19-30 %-wt vorgelegt.

Eine Übersicht der Festbettversuche und der entsprechenden Ergebnisse ist in Tabelle 2 zu sehen. Die Umsatzzuwächse sind generell im Bereich der Suspensionsversuche, aber die Verweilzeit hier ist wesentlich kürzer (ca. 90 min). Bei höheren CO₂-Förderraten werden deutlich höhere Umsatzraten mit einem Plus von bis zu 92 g/l DMC (Versuch 5) erzielt.

**Tabelle 4. Übersicht der Ergebnisse der Festbettversuche**

| **Nr.** | **Versuchsdauer** | **K-Salz im Bett (g)** | **DMC°** [%wt] | **MeOH°** [%wt] | **CO₂**° [g/min] | **τ** [min] | **Δ DMC [g/L]** |
|---|---|---|---|---|---|---|---|
| 1 | Tag 1 | 80 | 91 | 9 | 2,5 | 89 | 5 |
| | Tag 2 | 80 | 91 | 9 | 2,5 | 89 | 48 |
| | Tag 3 | 80 | 91 | 9 | 2,5 | 89 | 72 |

| **Regeneration des Festbetts durch Trocknen** | | | | | | | |
|---|---|---|---|---|---|---|---|
| 4 | Tag 4 nach Trocknen | 80 | 86 | 14 | 2,5 | 89 | 49 |

| **Neues Bett / höherer CO₂-Fluss** | | | | | | | |
|---|---|---|---|---|---|---|---|
| 5 | Tag 1 | 123 | 88 | 12 | 4 | 89 | 92 |

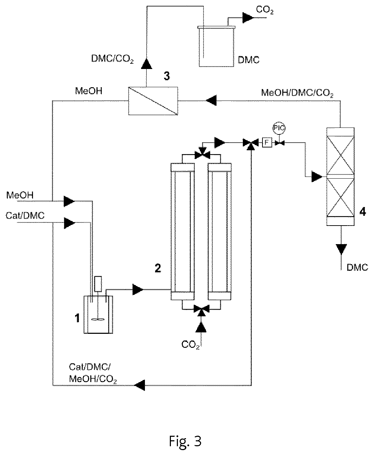

Die in den Figuren 1-3 dargestellten beiden Reaktoren werden abwechselnd ein- und ausgeschaltet, wenn sich herausstellt, dass das Festbett schon mit Wasser gesättigt ist, regeneriert bzw. getrocknet oder erneut werden muss.

### Beispiel 3: Behandlung des Reaktionsgemisches mittels Pervaporation

Ein Reaktionsgemisch, bestehend aus 75 %-wt Dimethylcarbonat, 20 %-wt Methanol und 5 %-wt Wasser, wird mittels einer Membranpervaporation-Anlage, ausgerüstet mit keramischen speziell beschichteten hydrophilen Membranen, bei einem Druck von 0-10 bar und einer Temperatur von 30-70°C bearbeitet. Nach der Separation enthält das Retentat nur noch 0,2 %-wt Wasser und 0,4 %-wt Methanol.

### Beispiel 4: Behandlung des Reaktionsgemisches mittels Dampfpermeation

Das Reaktionsgemisch, bestehend aus 75 %-wt Dimethylcarbonat, 20 %-wt Methanol und 5 %-wt Wasser, wird direkt in dampfförmigem Zustand durch hydrophile keramische Membranen getrennt. Die Feedtemperatur liegt vorzugsweise zwischen 90°C und 120 °C, der Dampf wird anschließend überhitzt auf 100°C bis 200°C. Das Retentat enthält danach noch 0,2 %-wt Wasser und 0,4 %-wt Methanol. Das Permeat enthält zwischen 95 %-wt und 98 %-wt Methanol. Die Trennung Methanol/Wasser erfolgt destillativ oder durch Einsatz von Trocknungsmitteln.

## Patentansprüche

1. Verfahren zur Herstellung organischer Carbonate (R¹O₂)CO durch katalysierte Reaktion von entsprechenden Alkoholen R¹OH mit Kohlendioxid, **dadurch gekennzeichnet, dass** die Ausgangsprodukte für die Reaktion neben ROH und Kohlendioxid auch das Endprodukt (R¹O₂)CO umfassen, wobei R¹ ein gerad- oder verzweigtkettiger Alkylrest mit 1-4 Kohlenstoffatomen ist und als Katalysatoren Verbindungen der Formel Alkali₂CO₃ verwendet werden, wobei Alkali ein Element ausgewählt aus der Gruppe Lithium, Natrium, Kalium, Rubidium, Cäsium darstellt, vorzugsweise K₂CO₃ und Cs₂CO₃ sowie Gemische aus den beiden, besonders bevorzugt K₂CO₃.

2. Verfahren nach Anspruch 1, **dadurch gekennzeichnet, dass** die Ausgangsprodukte 30 bis 90 %-wt, vorzugsweise 65 bis 85 %-wt, an Endprodukt (R¹O₂)CO umfassen.

3. Verfahren nach Anspruch 1 oder 2, **dadurch gekennzeichnet, dass** die basischen Katalysatoren zusätzlich zu ihrer katalytischen Wirkung auch wasseraufnehmend wirken.

4. Verfahren nach einem der Ansprüche 1 bis 3, **dadurch gekennzeichnet, dass** das Ausgangsprodukt CO₂ der Reaktion als Gas oder als Flüssigkeit zugeführt wird.

5. Verfahren nach einem der Ansprüche 1 bis 4, **dadurch gekennzeichnet, dass** die Reaktion kontinuierlich oder diskontinuierlich durchgeführt wird.

6. Verfahren nach einem der Ansprüche 1 bis 5 **dadurch gekennzeichnet, dass** die Reaktion in einem Strömungsreaktor oder in einer Serie von Reaktoren durchgeführt wird.

7. Verfahren nach einem der Ansprüche 1 bis 6, **dadurch gekennzeichnet, dass** die Reaktion bei einer Temperatur von 60-140°C, bevorzugt 90-110°C durchgeführt wird.

8. Verfahren nach einem der Ansprüche 1 bis 7, **dadurch gekennzeichnet, dass** die Reaktion bei einem Druck von 20-120 bar, bevorzugter 30-50 bar, am bevorzugtesten 70-90 bar durchgeführt wird.

9. Verfahren nach einem der Ansprüche 1 bis 8, **dadurch gekennzeichnet, dass** als Alkohol Methanol, Ethanol, Propanol oder Butanol eingesetzt wird.

10. Verfahren nach einem der Ansprüche 1 bis 9, **dadurch gekennzeichnet, dass** bei den Ausgangsprodukten die Menge an Katalysator im Verhältnis zum eingesetzten Alkohol 0,02-1,0 Moläquivalente beträgt.

11. Verfahren nach einem der Ansprüche 1 bis 10, **dadurch gekennzeichnet, dass** bei den Ausgangsprodukten die Menge an organischem Carbonat im Verhältnis zum eingesetzten Alkohol 0,5-5,0 Moläquivalente beträgt.

12. Verfahren nach einem der Ansprüche 1 bis 11, **dadurch gekennzeichnet, dass** bei den Ausgangsprodukten die Menge an CO₂ im Verhältnis zum eingesetzten Alkohol 0,5-7,5 Moläquivalente beträgt.

13. Verfahren nach einem der Ansprüche 1 bis 12, **dadurch gekennzeichnet, dass** die Abtrennung des Endprodukts durch Membranpervaporation oder Dampfpermeation oder als Hybridseparation in Kombination mit Destillation erfolgt.

14. Verfahren nach einem der Ansprüche 1 bis 13, **dadurch gekennzeichnet, dass** der eingesetzte Alkohol Methanol (MeOH) und das Endprodukt Dimethylcarbonat (DMC) ist.

15. Verfahren nach Anspruch 14, **dadurch gekennzeichnet, dass** als Ausgangsprodukte für die Reaktion DMC und MeOH in einem azeotropen Mischungsverhältnis vorliegen.

## Claims

1. Method for preparing organic carbonates (R¹O₂)CO by the catalysed reaction of suitable alcohols R¹OH with carbon dioxide, **characterised in that** the starting products for the reaction, in addition to ROH and carbon dioxide, also include the end product (R¹O₂)CO, where R¹ is a straight-chain or branched-chain alkyl functional group having from 1 to 4 carbon atoms, and compounds of the formula alkali₂CO₃ are used as catalysts, alkali being an element selected from the group lithium, sodium, potassium, rubidium, caesium, preferably K₂CO₃ and Cs₂CO₃ and mixtures of the two, particularly preferably K₂CO₃.

2. Method according to claim 1, **characterised in that** the starting products include from 30 to 90 wt.%, preferably from 65 to 85 wt.%, of end product (R¹O₂)CO.

3. Method according to either claim 1 or claim 2, **characterised in that** the basic catalysts, in addition to their catalytic effect, also act in a water-absorbing manner.

4. Method according to any of claims 1 to 3, **characterised in that** the starting product CO₂ of the reaction is supplied as a gas or as a liquid.

5. Method according to any of claims 1 to 4, **characterised in that** the reaction is carried out continuously or batchwise.

6. Method according to any of claims 1 to 5, **characterised in that** the reaction is carried out in a flow reactor or in a series of reactors.

7. Method according to any of claims 1 to 6, **characterised in that** the reaction is carried out at a temperature of from 60 to 140°C, preferably from 90 to 110°C.

8. Method according to any of claims 1 to 7, **characterised in that** the reaction is carried out at a pressure of from 20 to 120 bar, more preferably from 30 to 50 bar and most preferably from 70 to 90 bar.

9. Method according to any of claims 1 to 8, **characterised in that** methanol, ethanol, propanol or butanol is used as the alcohol.

10. Method according to any of claims 1 to 9, **characterised in that**, in the starting products, the amount of catalyst in relation to the alcohol used is of from 0.02 to 1.0 mol equivalents.

11. Method according to any of claims 1 to 10, **characterised in that**, in the starting products, the amount of organic carbonate in relation to the alcohol used is of from 0.5 to 5.0 mol equivalents.

12. Method according to any of claims 1 to 11, **characterised in that**, in the starting products, the amount of CO₂ in relation to the alcohol used is of from 0.5 to 7.5 mol equivalents.

13. Method according to any of claims 1 to 12, **characterised in that** the end product is separated by membrane pervaporation or vapour permeation or as a hybrid separation process in combination with distillation.

14. Method according to any of claims 1 to 13, **characterised in that** the alcohol used is methanol (MeOH) and the end product is dimethyl carbonate (DMC).

15. Method according to claim 14, **characterised in that**, as starting products for the reaction, DMC and MeOH are present in an azeotropic mixing ratio.

## Revendications

1. Procédé de préparation de carbonates organiques (R¹O₂)CO par une réaction catalysée d'alcools correspondants R¹OH avec du dioxyde de carbone, **caractérisé en ce que** les produits de départ servant à la réaction contiennent aussi, en plus du ROH et du dioxyde de carbone, le produit final (R¹O₂)CO, où R¹ est un radical alkyle à chaîne droite ou ramifiée ayant 1 à 4 atomes de carbone, et on utilise en tant que catalyseurs des composés de formule alcali₂CO₃, alcali étant un élément choisi dans le groupe consistant en le lithium, le sodium, le potassium, le rubidium, le césium, de préférence K₂CO₃ et Cs₂CO₃, ainsi que les mélanges des deux, d'une manière particulièrement préférée K₂CO₃.

2. Procédé selon la revendication 1, **caractérisé en ce que** les produits de départ comprennent 30 à 90 % en poids, de préférence 65 à 85 % en poids, du produit final (R¹O₂)CO.

3. Procédé selon la revendication 1 ou 2, **caractérisé en ce que** les catalyseurs basiques ont aussi, en plus de leur effet catalytique, un effet d'absorption de l'eau.

4. Procédé selon l'une des revendications 1 à 3, **caractérisé en ce que** le produit de départ CO₂ est amené à la réaction sous forme d'un gaz ou sous forme d'un liquide.

5. Procédé selon l'une des revendications 1 à 4, **caractérisé en ce que** la réaction est mise en oeuvre d'une manière continue ou discontinue.

6. Procédé selon l'une des revendications 1 à 5, **caractérisé en ce que** la réaction est mise en oeuvre dans un réacteur continu ou dans une série de réacteurs.

7. Procédé selon l'une des revendications 1 à 6, **caractérisé en ce que** la réaction est mise en oeuvre à une température de 60 à 140 °C, de préférence de 90 à 110 °C.

8. Procédé selon l'une des revendications 1 à 7, **caractérisé en ce que** la réaction est mise en oeuvre sous une pression de 20 à 120 bar, de préférence de 30 à 50 bar, idéalement de 70 à 90 bar.

9. Procédé selon l'une des revendications 1 à 8, **caractérisé en ce qu'**on utilise en tant qu'alcool du méthanol, de l'éthanol, du propanol ou du butanol.

10. Procédé selon l'une des revendications 1 à 9, **caractérisé en ce que**, dans le cas des produits de départ, la quantité du catalyseur, rapportée à l'alcool utilisé, est de 0,02 à 1,0 équivalents en moles.

11. Procédé selon l'une des revendications 1 à 10, **caractérisé en ce que**, dans le cas des produits de départ, la quantité du carbone organique, rapportée à l'alcool utilisé, est de 0,5 à 5,0 équivalents en moles.

12. Procédé selon l'une des revendications 1 à 11, **caractérisé en ce que**, dans le cas des produits de départ, la quantité de CO₂, rapportée à l'alcool utilisé, est de 0,5 à 7,5 équivalents en moles.

13. Procédé selon l'une des revendications 1 à 12, **caractérisé en ce que** la séparation du produit final s'effectue par pervaporation sur membrane ou par perméation de vapeur, ou sous forme d'une séparation hybride en combinaison avec une distillation.

14. Procédé selon l'une des revendications 1 à 13, **caractérisé en ce que** l'alcool utilisé est le méthanol (MeOH) et le produit final le carbonate de diméthyle (DMC).

15. Procédé selon la revendication 14, **caractérisé en ce que**, en tant que produit de départ pour la réaction, le DMC et le MeOH sont utilisés selon un rapport de mélange azéotropique.
